# EUROPÄISCHE PATENTSCHRIFT

(11) **EP 2 051 666 B1**
(45) Veröffentlichungstag und Bekanntmachung des Hinweises auf die Patenterteilung: **26.10.2011**
(21) Anmeldenummer: 07787757.9
(22) Anmeldetag: 20.07.2007
(51) Int. Cl.: A61F 2/30, A61L 27/30, C23C 28/00

(54) **IMPLANTAT UND VERFAHREN ZUM HERSTELLEN EINES IMPLANTATS**
IMPLANT, AND METHOD FOR THE PRODUCTION OF AN IMPLANT
IMPLANT ET PROCÉDÉ DE PRODUCTION D'UN IMPLANT

(30) Priorität: 16.08.2006 DE 102006039329
(43) Veröffentlichungstag der Anmeldung: 29.04.2009
(73) Patentinhaber: Ionbond AG Olten, 4600 Olten (CH); Aesculap AG, 78532 Tuttlingen (DE)
(72) Erfinder: ZELLER, Richard, 78532 Tuttlingen (DE); REICH, Jan, 78073 Hochemmingen (DE); GROHMANN, Fred-Rainer, 8902 Urdorf (CH)
(74) Vertreter: Hoeger, Stellrecht & Partner Patentanwälte
(86) Internationale Anmeldenummer: PCT/EP2007/057502
(87) Internationale Veröffentlichungsnummer: WO 2008/019923

(56) Entgegenhaltungen:
- EP-A- 0 248 117
- EP-A- 1 679 088
- WO-A-2006/069465
- DE-A1- 4 012 048
- DE-U1-202005 005 405

## Beschreibung

Die vorliegende Erfindung betrifft ein Implantat zum Einsetzen in einen menschlichen oder tierischen Körper, welches mindestens ein Implantatteil umfasst, wobei das mindestens eine Implantatteil einen aus einem Implantatmaterial hergestellten Grundkörper mit einer Oberfläche aufweist, die mindestens teilweise in Form einer künstlichen Gelenkfläche ausgebildet ist, wobei ein Teil oder die ganze Gelenkfläche mit einer verschleißmindernden Hartstoffbeschichtung bedeckt ist, wobei zwischen der Hartstoffbeschichtung und der mindestens einen aus dem Implantatmaterial gebildeten Gelenkfläche eine Zwischenschicht zum Vermindern von Spannungen zwischen der Hartstoffbeschichtung und dem Implantatmaterial vorgesehen ist und wobei die Hartstoffbeschichtung in Form eines mehrschichtigen Schichtsystems ausgebildet ist.

Ferner betrifft die vorliegende Erfindung ein Verfahren zum Herstellen eines Implantats zum Einsetzen in einen menschlichen oder tierischen Körper, welches mindestens ein Implantatteil umfasst, wobei zur Ausbildung des Implantatteils ein Grundkörper aus einem Implantatmaterial hergestellt wird, welches eine Oberfläche aufweist, die mindestens in Form einer künstlichen Gelenkfläche ausgebildet wird, wobei eine verschleißmindernde Hartstoffbeschichtung auf die ganze oder einen Teil der Gelenkfläche aufgebracht wird, wobei vor dem Aufbringen der Hartstoffbeschichtung eine Zwischenschicht zum Vermindern mechanischer Spannungen zwischen der Hartstoffbeschichtung und dem Implantatmaterial auf dem mindestens einen Teil der Gelenkfläche aufgebracht wird und wobei die Hartstoffbeschichtung in Form eines mehrschichtigen Schichtsystems ausgebildet wird.

Implantate der eingangs beschriebenen Art und Verfahren zu deren Herstellung sind in vielfältiger Weise bekannt. Derartige Implantate kommen beispielsweise in Form von Hüft- und Kniegelenk-Endoprothesen zum Einsatz. Ein weiteres Anwendungsgebiet ist die Wirbelsäulenprothetik, dabei insbesondere die Bandscheibenprothetik. Unabhängig von ihrem Einsatzzweck weisen Implantate der eingangs beschriebenen Art eine Gelenkfläche auf, die vorzugsweise verschleißmindernd ausgebildet ist. Zu diesem Zweck ist es bekannt, einen ein Implantatteil bildenden Grundkörper mit einer Hartstoffbeschichtung zu versehen, und zwar mindestens in einem Bereich seiner Oberfläche, welche einen Teil oder die gesamte Gelenkfläche bildet.

Bei bekannten Implantaten tritt jedoch üblicherweise das Problem auf, dass aus dem Implantatmaterial Metallionen, beispielsweise Kobalt-, Chrom-, Molybdän- und/oder Nickel-Ionen austreten können, und zwar auch durch die Hartstoffbeschichtung hindurch. Dies führt zu einer erhöhten Korrosion und auch zu einer Erhöhung des Verschleißes des Implantats, beispielsweise durch Abplatzen der Hartstoffbeschichtung. Die beschriebenen negativen Effekte haben den Nachteil, dass sich dadurch eine Implantattragedauer sowie die Verträglichkeit des Implantats reduzieren kann, beispielsweise kann der Austritt von Metallionen im großen Umfang aus dem Implantatmaterial allergische Reaktionen hervorrufen.

Aus der EP 1 679 088 A2 ist eine medizinische Vorrichtung bekannt, die mit einem nanostrukturierten Material beschichtet ist. Die WO 2006/069465 A1 offenbart ein Prothesengelenk mit Gelenkflächenschichten umfassend amorphen, diamantähnlichen Kohlenstoff (ADLC).

Es ist daher Aufgabe der vorliegenden Erfindung, ein Implantat der eingangs beschriebenen Art sowie ein Verfahren zur Herstellung eines Implantats so zu verbessern, dass eine Implantattragedauer verlängert und die Verträglichkeit des Implantats erhöht wird.

Diese Aufgabe wird durch ein Implantat der eingangs beschriebenen Art erfindungsgemäß dadurch gelöst, dass das Schichtsystem eine vom Implantatteil weg weisende äußere Deckschicht und mindestens eine unter der Deckschicht liegende innere Schicht aufweist, dass eine Härte der Deckschicht größer ist als eine Härte der mindestens einen inneren Schicht und dass die mindestens eine innere Schicht mindestens eine Chromnitrid-(CrN)-Schicht oder mindestens eine Chromcarbonitrid-(CrCN)-Schicht umfasst.

Die erfindungsgemäß vorgeschlagene Zwischenschicht hat insbesondere die Eigenschaft, mechanische und/oder elektromechanische Spannungen zwischen der Hartstoffbeschichtung und dem Implantatmaterial und/oder innere Spannungen in der Hartstoffbeschichtung zu vermindern. Dies hat den Vorteil, dass die Hartstoffbeschichtung praktisch ohne mechanische und/oder elektromechanische Spannungen auf das Implantatmaterial aufgebracht werden kann. Dadurch wird es Metallionen, die aus dem Implantatmaterial austreten möchten, erschwert, sowohl durch die Zwischenschicht als auch durch die Hartstoffbeschichtung nach außen vorzudringen. Dadurch wird einerseits eine Korrosion des Implantatmaterials verringert und andererseits ein Verschleiß der Hartstoffbeschichtung reduziert. Insbesondere kann die Zwischenschicht auch ein Anhaften der Hartstoffbeschichtung auf dem Implantatmaterial verbessern. Insbesondere dann, wenn die Zwischenschicht eine defektarme Schicht ist, bildet diese Kondensationskeime für ein optimales Aufwachsen der Hartstoffbeschichtung. Vorteilhafterweise ist die Hartstoffbeschichtung in Form eines mehrschichtigen Schichtsystems ausgebildet. Dadurch können Eigenschaften der Hartstoffbeschichtung individuell optimiert werden. So kann zum einen eine erforderliche Stützwirkung für eine äußere Deckschicht verbessert und zum anderen ein Abplatzen dieser Deckschicht wirksam verhindert werden. Ein mehrschichtiges Schichtsystem, das beispielsweise in Form einer "Sandwich-Struktur" aufgebaut sein kann, verbessert zudem die Ermüdungsfestigkeit des Schichtsystems selbst. Des Weiteren können durch mehrere unterschiedliche Schichten gezielt Barrieren für Metallionen ausgebildet werden. Günstig ist es, dass das Schichtsystem eine vom Implantatteil weg weisende äußere Deckschicht und mindestens eine unter der Deckschicht liegende innere Schicht aufweist und dass eine Härte der Deckschicht größer ist als eine Härte der mindestens einen inneren Schicht. So kann beispielsweise die äußere Deckschicht deutlich härter sein als die mindestens eine darunter liegende Schicht, die jedoch vorzugsweise eine ausreichende Zähigkeit aufweist, um sowohl eine Ermüdungsfestigkeit des Schichtsystems zu erhöhen als auch ein Abplatzen der äußeren Deckschicht zu verhindern. Vorzugsweise umfasst die mindestens eine innere Schicht mindestens eine Chromnitrid-(CrN)-Schicht. Diese harte Schicht erhöht eine Stützwirkung des Schichtsystems für die äußere Deckschicht. Günstig ist es ferner, wenn die mindestens eine innere Schicht mindestens eine Chromcarbonitrid-(CrCN)-Schicht umfasst. Die Kohlenstoff enthaltende Chromcarbonitrid-(CrCN)-Schicht weist eine große Zähigkeit auf, die insbesondere ein Abplatzen weiterer Schichten, vor allem der äußeren Deckschicht, verhindert.

Vorzugsweise ist die Hartstoffbeschichtung in Form einer korrosionsinhibierenden Beschichtung ausgebildet. Durch eine derartige Hartstoffbeschichtung wird sowohl eine Korrosion des Implantatmaterials nach der Implantation in einen menschlichen oder tierischen Körper als auch eine Korrosion der Hartstoffbeschichtung selbst deutlich verringert oder sogar ganz verhindert.

Um zu verhindern, dass Metallionen aus dem Implantatmaterial austreten und in den menschlichen oder tierischen Körper gelangen können, ist es günstig, wenn die Hartstoffbeschichtung in Form einer eine Metallionen-Barriere bildenden Beschichtung ausgebildet ist.

Vorteilhaft ist es, wenn die eine Metallionen-Barriere bildende Beschichtung eine die Ionenabgabe aus dem Implantatteil im Vergleich zu einem Implantatteil ohne eine Metallionen-Barriere bildende Beschichtung um mindestens einen Faktor 10 verringernde Beschichtung ist, vorzugsweise eine die Ionenabgabe um mindestens einen Faktor 20 verringernde Beschichtung. Mit einer derartigen eine Metallionen-Barriere bildenden Beschichtung kann der Austritt von Metallionen aus dem Implantatmaterial im Vergleich zu herkömmlichen Implantaten auf mindestens ein Zehntel verringert werden.

Günstigerweise ist die eine Metallionen-Barriere bildende Beschichtung eine Barriere für Kobalt-, Chrom-, Molybdän- und/oder Nickel-Ionen. Eine solche Metallionen-Barriere bildende Beschichtung eignet sich hervorragend für herkömmliche Implantatwerkstoffe, beispielsweise Stähle, die Kobalt, Chrom, Molybdän und/oder Nickel enthalten.

Die Herstellung der Hartstoffbeschichtung wird besonders einfach und deren Verschleißfestigkeit besonders hoch, wenn die Hartstoffbeschichtung mindestens eine keramische Schicht umfasst.

Günstig ist es, wenn die Deckschicht Zirkon (Zr) enthält, denn Zirkon enthaltende Schichten oder Keramiken weisen eine besonders große Härte und damit eine hohe Abriebfestigkeit auf.

Um die Verschleißminderung der Hartstoffbeschichtung weiter zu verbessern, ist es vorteilhaft, wenn die Deckschicht Zirkonnitrid (ZrN) ist. Neben der hohen Härte bewirkt ein geänderter Benetzungswinkel der Zirkonnitrid-(ZrN)-Schicht eine Verbesserung der Schmierung der Gleitflächen und trägt somit zu einer weiteren Verschleißreduzierung bei.

Vorzugsweise sind mehrere innere Schichten ausgebildet. Mehrere innere Schichten, vorzugsweise in Form einer "Sandwich-Struktur", erhöhen eine Stützwirkung für die Deckschicht und verhindern in hohem Maße deren Abplatzen.

Zur Erhöhung einer Härte und der Stabilität der Hartstoffbeschichtung ist es vorteilhaft, wenn die mindestens eine innere Schicht eine Nitrid-Schicht ist. Als Nitrid-Schichten sind insbesondere Schichten aus Titannitrid (TiN), Titancarbonitrid (TiCN), Chromnitrid (CrN) und Chromcarbonitrid (CrCn) denkbar.

Um definiert unterschiedliche Eigenschaften des Schichtsystems vorgeben zu können, ist es günstig, wenn mindestens zwei unterschiedliche innere Schichten ausgebildet sind. Selbstverständlich können auch drei, vier oder mehr unterschiedliche innere Schichten vorgesehen sein.

Um ein hoch effizientes inneres Schichtsystem ausbilden zu können, ist es vorteilhaft, wenn mehr als zwei innere Schichten ausgebildet sind und wenn die mindestens zwei unterschiedlichen inneren Schichten abwechselnd übereinander liegend ausgebildet sind. Beispielsweise lässt sich so ein inneres Schichtsystem aus inneren Schichten unterschiedlicher Härte und Zähigkeit ausbilden, beispielsweise durch abwechselnde Schichten großer Härte und großer Zähigkeit.

Vorzugsweise sind fünf innere Schichten ausgebildet. Insbesondere können diese zwei unterschiedliche Schichten umfassen, die abwechselnd übereinander liegend ausgebildet sind, also zum Beispiel in einer Schichtfolge A-B-A-B-A. Insbesondere kann eine der beiden Schichten eine Chromnitrid-(CrN)-Schicht sein und die andere der beiden unterschiedlichen Schichten eine Chromcarbonitrid-(CrCN)-Schicht, wobei vorzugsweise die äußeren Schichten des Schichtsystems durch Chromnitrid-(CrN)-Schichten gebildet sind. Fünf innere Schichten lassen sich zum einen noch einfach herstellen, zum anderen kann bereits mit fünf Schichten eine sehr gute Haftung der äußeren Deckschicht sowie eine effiziente Metallionen-Barriere ausgebildet werden. Alternativ können als unterschiedliche Schichten auch Titannitrid-(TiN)-Schichten und Titancarbonitrid-(TiCN)-Schichten vorgesehen sein.

Um eine besonders gute Anhaftung der äußeren Deckschicht an der mindestens einen inneren Schicht zu gewährleisten, ist es vorteilhaft, wenn die Deckschicht direkt eine innere Schicht aus Chromnitrid (CrN) bedeckt. Insbesondere dann, wenn die äußere Deckschicht auch eine Nitrid-Schicht ist, kann so eine hervorragende Stabilität der Hartstoffbeschichtung erreicht werden.

Vorteilhafterweise ist die Zwischenschicht eine metallische Schicht. Eine metallische Schicht lässt sich besonders einfach auf ein metallisches Implantatmaterial aufbringen. Zudem haftet eine metallische Zwischenschicht besonders gut auf einem metallischen Implantatmaterial.

Vorzugsweise enthält die Zwischenschicht kein Metall, welches im Implantatmaterial enthalten ist. Eine derartige Zwischenschicht bildet eine zusätzliche Barriere für aus dem Implantatmaterial austretende Metallionen.

Gemäß einer bevorzugten Ausführungsform der Erfindung kann vorgesehen sein, dass die Zwischenschicht eine Schicht aus einer Zirkon-Legierung, aus einer Niob-Legierung, aus einer Tantal-Legierung, aus reinem Niob, aus reinem Tantal oder aus reinem Zirkon ist. Insbesondere eine Reinzirkon-Schicht, die vorteilhafterweise durch physikalische Gasphasenabscheidung (PVD) aufgebracht ist, ist besonders defektarm, so dass mechanische Spannungen, die aufträten, wenn die Hartstoffbeschichtung direkt auf das Implantatmaterial aufgebracht würde, deutlich verringert werden. Eine Schicht aus reinem Zirkon hat zudem den Vorteil, dass sie nahezu defektfrei ist, das heißt auch keine sogenannten "pin-holes" aufweist, durch die Ladungen und insbesondere auch Ionen aus dem Implantatmaterial in die Hartstoffbeschichtung oder durch diese hindurch gelangen können.

Ein besonders gutes Anhaften der Hartstoffbeschichtung und/oder der Zwischenschicht wird erreicht, wenn die Hartstoffbeschichtung und/oder die Zwischenschicht durch physikalische Gasphasenabscheidung (physical vapour deposition - PVD) aufgebrachte Schichten sind. Insbesondere lassen sich so mehrere Schichten nacheinander gezielt aufbringen.

Vorteilhafterweise weist die Zwischenschicht eine Dicke in einem Bereich von 30.10⁻⁹ m bis 200.10⁻⁹ m auf. Bevorzugt beträgt die Dicke der Schicht 50 bis 100 nm. Insbesondere eine Schicht aus metallischem Zirkonium hat ausgezeichnete Hafteigenschaften und versiegelt zudem das Implantatmaterial, wodurch ein Austritt von Metallionen aus dem Implantatmaterial reduziert werden kann.

Um eine Tragedauer des Implantats zu erhöhen, ist es vorteilhaft, wenn die Hartstoffbeschichtung eine Kratzfestigkeit von mindestens 100 N aufweist. Auf diese Weise lässt sich ein Abrieb der Hartstoffbeschichtung minimieren.

Vorzugsweise enthält das Implantatmaterial Kobalt oder ist eine Kobalt-Legierung. Es ist somit möglich, herkömmliche Implantatmaterialien zur Herstellung eines erfindungsgemäßen Implantats zu verwenden.

Vorteilhaft ist es, wenn die Kobalt-Legierung eine Kobalt-Chrom-Molybdän-Legierung ist. Vorzugsweise handelt es sich dabei um eine CoCr29Mo6-Legierung.

Gemäß einer bevorzugten Ausführungsform der Erfindung kann vorgesehen sein, dass das Implantat ein künstliches Hüft- oder Kniegelenk, ein Wirbelkörperersatzimplantat oder eine künstliche Bandscheibenprothese ist.

Vorzugsweise ist das mindestens eine Implantatteil eine Gelenkkugel oder eine Gelenkpfanne einer Hüftgelenkprothese, eine künstliche Kondyle oder eine Tibiaplatte einer Kniegelenkprothese oder ein eine Gelenkfläche aufweisendes oder tragendes Anlageelement einer Bandscheibenprothese.

Die eingangs gestellte Aufgabe wird ferner durch ein Verfahren der eingangs beschriebenen Art erfindungsgemäß dadurch gelöst, dass das Schichtsystem eine vom Implantatteil weg weisende äußere Deckschicht und mindestens eine unter der Deckschicht liegende innere Schicht aufweist, dass eine Härte der Deckschicht größer ist als eine Härte der mindestens einen inneren Schicht und dass die mindestens eine innere Schicht mindestens eine Chromnitrid-(CrN)-Schicht oder mindestens eine Chromcarbonitrid-(CrCN)-Schicht umfasst.

Wie bereits oben ausgeführt, wird durch die Zwischenschicht der Austritt von Metallionen aus dem Implantatmaterial deutlich herabgesetzt und ferner auch das Anhaften der Hartstoffbeschichtung auf dem' Implantatmaterial verbessert. Dadurch kann die Gefahr der Korrosion des Implantats vermindert und die Implantattragedauer erhöht werden. Zudem können Implantate auch für Allergiker aus Materialien hergestellt werden, die aufgrund sonst üblicherweise austretender, eine allergische Reaktion hervorrufender Materialien nicht zur Herstellung von Implantaten für Allergiker geeignet wären. Insbesondere durch eine defektarme Zwischenschicht lassen sich die gewünschten Eigenschaften der Hartstoffbeschichtung deutlich verbessern. Vorteilhafterweise ist die Hartstoffbeschichtung in Form eines mehrschichtigen Schichtsystems ausgebildet. Dadurch können Eigenschaften der Hartstoffbeschichtung individuell optimiert werden. So kann zum einen eine erforderliche Stützwirkung für eine äußere Deckschicht verbessert und zum anderen ein Abplatzen dieser Deckschicht wirksam verhindert werden. Ein mehrschichtiges Schichtsystem, das beispielsweise in Form einer "Sandwich-Struktur" aufgebaut sein kann, verbessert zudem die Ermüdungsfestigkeit des Schichtsystems selbst. Des Weiteren können durch mehrere unterschiedliche Schichten gezielt Barrieren für Metallionen ausgebildet werden. Günstig ist es, dass das Schichtsystem eine vom Implantatteil weg weisende äußere Deckschicht und mindestens eine unter der Deckschicht liegende innere Schicht aufweist und dass eine Härte der Deckschicht größer ist als eine Härte der mindestens einen inneren Schicht. So kann beispielsweise die äußere Deckschicht deutlich härter sein als die mindestens eine darunter liegende Schicht, die jedoch vorzugsweise eine ausreichende Zähigkeit aufweist, um sowohl eine Ermüdungsfestigkeit des Schichtsystems zu erhöhen als auch ein Abplatzen der äußeren Deckschicht zu verhindern. Vorzugsweise umfasst die mindestens eine innere Schicht mindestens eine Chromnitrid-(CrN)-Schicht. Diese harte Schicht erhöht eine Stützwirkung des Schichtsystems für die äußere Deckschicht. Günstig ist es ferner, wenn die mindestens eine innere Schicht mindestens eine Chromcarbonitrid-(CrCN)-Schicht umfasst. Die Kohlenstoff enthaltende Chromcarbonitrid-(CrCN)-Schicht weist eine große Zähigkeit auf, die insbesondere ein Abplatzen weiterer Schichten, vor allem der äußeren Deckschicht, verhindert.

Damit Schichten nahezu beliebiger Art und Dicke auf das Implantatteil aufgebracht werden können, ist es günstig, wenn die Hartstoffbeschichtung und/oder die Zwischenschicht durch physikalische Gasphasenabscheidung (physical vapour deposition - PVD) aufgebracht werden.

Um eine optimale Verbindung zwischen der Hartstoffbeschichtung und dem Implantatmaterial zu erreichen, ist es vorteilhaft, wenn die Zwischenschicht mit einer Dicke in einem Bereich von 30.10⁻⁹ m bis 200.10⁻⁹ m aufgebracht wird. Vorzugsweise wird die Zwischenschicht mit einer Dicke im Bereich von 50 bis 100 nm aufgebracht. Eine Zwischenschicht mit einer Dicke im angegebenen Bereich reicht aus, um das Implantatmaterial zu versiegeln, insbesondere dann, wenn die Zwischenschicht sehr defektarm ist, wie beispielsweise eine Zwischenschicht aus reinem Zirkonium.

Vorteilhafterweise wird als Zwischenschicht eine metallische Schicht aufgebracht. Insbesondere eignet sich eine Schicht aus reinem Zirkonium hervorragend um das Implantatmaterial zu versiegeln und eine optimale Verbindung zwischen der Hartstoffbeschichtung mit dem Implantatmaterial zu erreichen. Um eine möglichst gute, für Metallionen eine Barriere bildende Schicht auszubilden, ist es günstig, wenn als Zwischenschicht eine Schicht aufgebracht wird, die kein Metall enthält, welches im Implantatmaterial enthalten ist. Vorzugsweise wird als Zwischenschicht eine Schicht aus einer Zirkon-Legierung, aus einer Zirkon enthaltenden Keramik oder aus reinem Zirkon aufgebracht. Derartige Zwischenschichten eignen sich besonders gut, um das Implantatmaterial vollständig zu versiegeln.

Ferner wird die eingangs gestellte Aufgabe gelöst durch die Verwendung eines der oben beschriebenen Verfahren zur Herstellung eines Implantats in Form eines künstlichen Hüft- oder Kniegelenks, eines Wirbelkörperersatzimplantats oder einer künstlichen Bandscheibenprothese.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht eines Femurknochens mit einer implantierten erfindungsgemäßen Kondyle;
- Figur 2:: eine Ansicht des Femurknochens aus Figur 1 in Richtung des Pfeiles A;
- Figur 3:: eine Ansicht eines teilweise künstlichen Kniegelenks von vorne;
- Figur 4:: eine Schnittansicht längs der Linien 4-4 in den Figuren 1 und 3; und
- Figur 5:: eine Ansicht analog Figur 4 mit einem mehrschichtigen Hartstoffbeschichtungssystem.

Erfindungsgemäße Implantate können beispielsweise in Form von Kniegelenkprothesen, Hüftgelenkprothesen oder Bandscheibenprothesen ausgebildet sein. Diese Liste ist jedoch nicht abschließend.

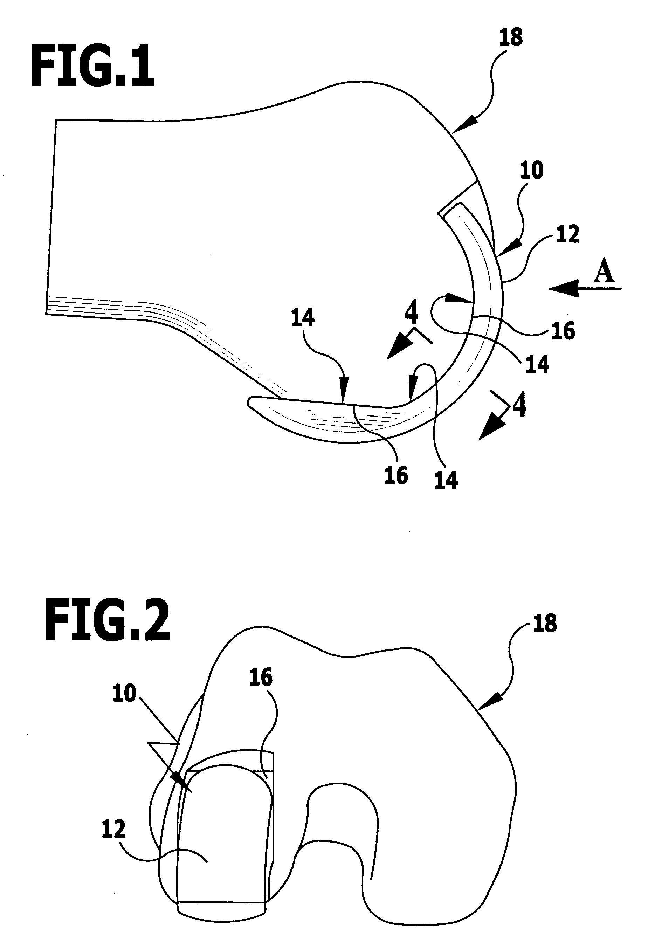

Im Falle einer Kniegelenkprothese ist es denkbar, einen mit einem Femur 18 verbindbaren Teil der Kniegelenkprothese in Form eines Implantatteils gemäß der vorliegenden Erfindung auszubilden, beispielsweise in Form eines Implantatteils 10, wie er beispielhaft in den Figuren 1 und 2 in Form einer künstlichen Kondyle dargestellt ist. Das Implantatteil 10 weist eine Artikulationsfläche auf, die eine Gelenkfläche 12 bildet. Eine in die entgegengesetzte Richtung weisende Knochenanlagefläche 14 des Implantatteils 10 liegt an einer bearbeiteten Knochenfläche 16 des Femurs 18 an.

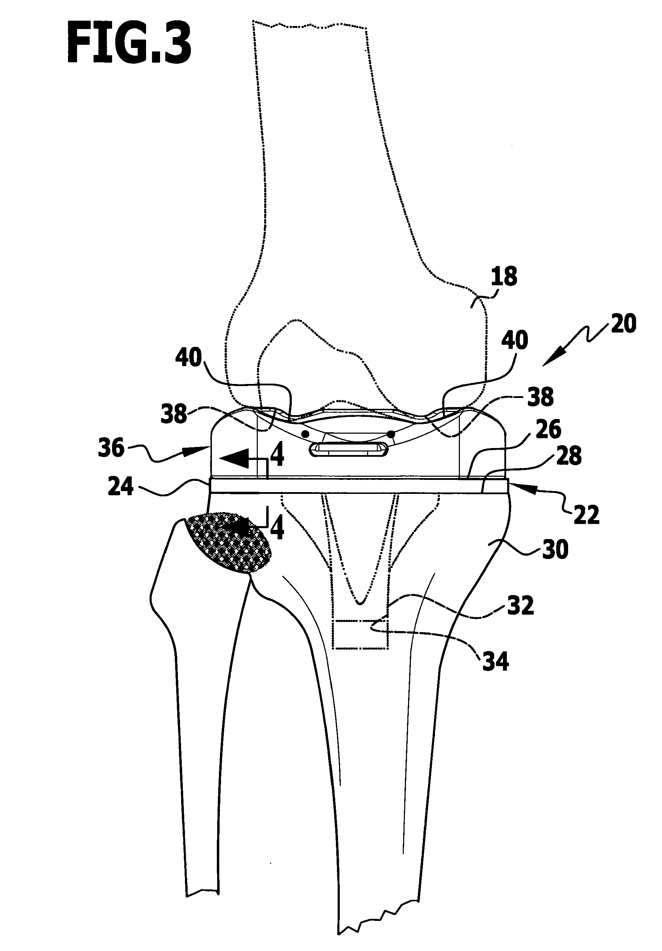

Des Weiteren kann zur Ausbildung eines Implantats 20 in Form einer Kniegelenkprothese ein weiteres erfindungsgemäßes Implantatteil vorgesehen sein, welches in Figur 3 mit dem Bezugszeichen 22 versehen und in Form eines Tibiateils ausgebildet ist. Das Implantatteil 22 umfasst eine Tibiaplatte 24 mit einer ebenen Gelenkfläche 26, die an einer vorbereiteten ebenen Knochenfläche 28 einer Tibia 30 anliegt. Das Implantatteil 22 weist ferner einen Schaft 32 auf, welcher in einer dafür vorbereiteten Ausnehmung 34 in der Tibia verankert ist.

Das Implantatteil 20 kann ferner einen Meniskusteil 36 umfassen, welcher beweglich auf dem Implantatteil 22 gelagert ist. Der Meniskusteil 36 weist zwei zu Kondylen 38 des Femurs korrespondierende Artikulationsflächen 40 auf. Insbesondere können die Artikulationsflächen 40 korrespondierend zu der Gelenkfläche 12 des Implantatteils 10 ausgebildet sein.

Die Gelenkfläche 12 des Implantatteils 10 und die Gelenkfläche 26 des Implantatteils 22 sind mindestens teilweise, vorzugsweise vollständig, mit einer

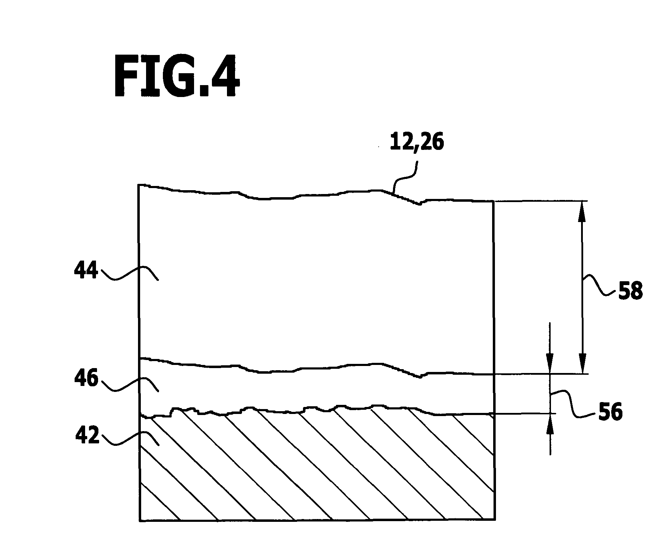

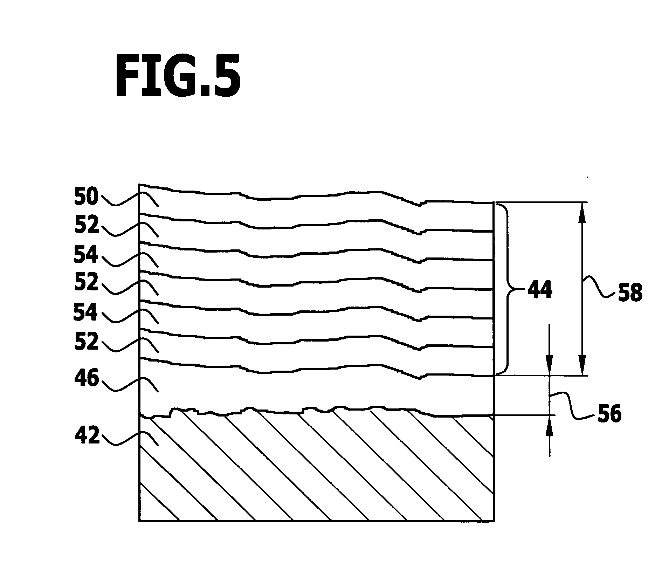

Hartstoffbeschichtung versehen. Ein Grundkörper 42 der Implantatteile 10 und 22, welcher vorzugsweise aus einem Implantatstahl, beispielsweise einer Kobalt-Chrom-Molybdän-Legierung, wie zum Beispiel CoCr29Mo6, hergestellt ist, ist, wie in den Figuren 4 und 5 beispielhaft dargestellt, mit einer Hartstoffbeschichtung 44 versehen.

Zwischen dem Grundkörper 42 und der Hartstoffbeschichtung 44 ist eine Zwischenschicht 46 aufgebracht, die mechanische Spannungen zwischen der Hartstoffbeschichtung 44 und dem Implantatmaterial, aus dem der Grundkörper 42 gebildet ist, vermindert.

Die Hartstoffbeschichtung 44 kann in Form einer einzigen Schicht aufgebracht sein oder aber auch in Form eines Schichtsystems, welches aus zwei oder mehr Schichten aufgebaut ist. Ein aus mehreren Schichten bestehendes Schichtsystem umfasst insbesondere eine äußere Deckschicht 50, die vorzugsweise in Form einer verschleißtragenden Zirkonnitrid-Schicht (ZrN) ausgebildet ist. Sie weist vorzugsweise eine Dicke in einem Bereich von 1·10 ⁶m bis 10·10⁻⁶ m auf.

Unter der Deckschicht 50 können eine oder mehrere innere Schichten des Schichtsystems vorgesehen sein. Vorzugsweise sind die inneren Schichten in Form von zähen und harten Schichten 52 beziehungsweise 54 ausgebildet, die insbesondere abwechselnd übereinander liegend das Schichtsystem ausbilden können. Als ein Beispiel für eine harte innere Schicht 52 sei eine Chromnitrid-(CrN)-Schicht genannt, als zähe Schichten 54 eignen sich insbesondere Schichten aus Chromcarbonitrid (CrCN).

Besonders gute Eigenschaften der Hartstoffbeschichtung 44 werden erreicht, wenn abwechselnd harte und zähe Schichten vorgesehen sind. Sie geben der äußeren Deckschicht die erforderliche Stützwirkung und verhindern in hohem Maße deren Abplatzen. Ferner wird durch eine derartige "Sandwich-Struktur" auch die Ermüdungsfestigkeit des Schichtsystems maßgeblich positiv beeinflusst. Die Deckschicht, beispielsweise eine Zirkonnitrid-(ZrN)-Deckschicht ist in ihrer Härte auf die Härte der harten Schichten 52 abgestimmt und übernimmt im Wesentlichen die verschleißmindernde Funktion der Hartstoffbeschichtung 44. Neben der hohen Härte hat sich bei einer Zirkonnitrid-(ZrN)-Deckschicht überraschend gezeigt, dass bei dieser Schicht insbesondere ein geänderter Benetzungswinkel eine Verbesserung der Schmierung der Gelenkfläche 12 beziehungsweise der Gelenkfläche 26 bewirkt und dadurch zu einer Verschleißreduzierung beträgt.

Die Zwischenschicht 46, die vorzugsweise aus reinem Zirkonium ausgebildet ist, ist praktisch defektfrei, das heißt es werden keine sogenannten "pin-holes" ausgebildet, die einen Ladungstransport, insbesondere auch in Form von Metallionen, aus dem Grundkörper 42 nach außen ermöglichen.

Die Hartstoffbeschichtung 44, insbesondere wenn sie in Form eines Schichtsystems ausgebildet ist, wirkt sich nicht nur positiv auf die Stabilität aus, sondern verhindert zudem eine Abgabe von Metallionen aus dem Grundkörper 42. Ein Austreten von Metallionen aus einem Implantatteil 10 beziehungsweise 22 kann bei manchen Patienten zu allergischen Reaktionen führen und ist keinesfalls wünschenswert. Insbesondere das vorgeschlagene Schichtsystem ermöglicht es, eine Ionenabgabe von Kobalt, Chrom, Molybdän und/oder
Nickel aus dem Implantatmaterial des Grundkörpers 42 um das mindestens 20fache gegenüber herkömmlichen Implantaten zu reduzieren. Durch die vorzugsweise defektfreie Zwischenschicht 46 wird die Ionenabgabe zusätzlich verringert. Insbesondere ist es von Vorteil, wenn die Zwischenschicht 46 aus einem Metall gebildet ist, welches nicht im Implantatmaterial enthalten ist, aus dem der Grundkörper 42 hergestellt ist.

Die Hartstoffbeschichtung 44 beziehungsweise die einzelnen Schichten derselben werden vorzugsweise mittels physikalischer Gasphasenabscheidung aufgebracht, bilden also sogenannte "PVD-Schichten (physical vapour deposition)". Die Schichten könnten alternativ auch durch Kaltgasspritzen aufgebracht werden. Die Zwischenschicht weist vorzugsweise eine Dicke in einem Bereich von 50·10⁻⁹ m bis 100·10⁻⁹ m auf, die Hartstoffbeschichtung 44 eine Gesamtdicke 58 in einem Bereich von 1·10⁻⁶ m bis 10·10⁻⁶ m. Dicken der einzelnen Schichten des Schichtsystems, insbesondere der harten Schicht 52 und der zähen Schicht 54 liegen in einem Bereich von 200·10⁻⁹ m bis 1000·10⁻⁹ m. Die Deckschicht 50 weist vorzugsweise eine Dicke in einem Bereich von 1·10⁻⁶ m bis 5·10⁻⁶ m auf.

Insbesondere durch das Multilagen-Schichtsystem ist es gelungen, die bekannten guten Verschleißschutzeigenschaften von Hartstoffschichten für das tribologische, korrosive und dynamische Beanspruchungsprofil bei Gelenkprothesen, insbesondere bei Kniegelenk-Endoprothesen, zu adaptieren. Die einzelnen Schichten besitzen Barrierefunktion und gleichzeitig bedingt der Härtegradient der Schichten und die gradierte Druckspannungsverteilung der Einzelschichten eine hohe Haftfestigkeit und eine exzellente Ermüdungsfestigkeit. Insbesondere eine Gradierung des Kohlenstoffs innerhalb der zähen Schichten 54, beispielsweise innerhalb von Chromcarbonitrid-(CrCN)-Schichten, hat gezeigt, dass so eine Druckeigenspannungsverteilung aufbaut werden kann, so dass eine Kratzfestigkeit der Hartstoffbeschichtung 44 insgesamt über 100 N liegt. Typische bekannte PVD-Schichten weisen eine Kratzfestigkeit lediglich in einem Bereich zwischen 50 N und 70 N auf.

## Patentansprüche

1. Implantat (20) zum Einsetzen in einen menschlichen oder tierischen Körper, welches mindestens ein Implantatteil (10, 22) umfasst, wobei das mindestens eine Implantatteil (10, 22) einen aus einem Implantatmaterial hergestellten Grundkörper (42) mit einer Oberfläche aufweist, die mindestens teilweise in Form einer künstlichen Gelenkfläche (12, 26) ausgebildet ist, wobei ein Teil der oder die ganze Gelenkfläche (12, 26) mit einer verschleißmindernden Hartstoffbeschichtung (44) bedeckt ist, wobei zwischen der Hartstoffbeschichtung (44) und der mindestens einen aus dem Implantatmaterial gebildeten Gelenkfläche (12, 26) eine Zwischenschicht (46) zum Vermindern von Spannungen zwischen der Hartstoffbeschichtung (44) und dem Implantatmaterial vorgesehen ist und wobei die Hartstoffbeschichtung (44) in Form eines mehrschichtigen Schichtsystems ausgebildet ist, **dadurch gekennzeichnet, dass** das Schichtsystem eine vom Implantatteil (10, 22) weg weisende äußere Deckschicht (50) und mindestens eine unter der Deckschicht (50) liegende innere Schicht (52, 54) aufweist, dass eine Härte der Deckschicht (50) größer ist als eine Härte der mindestens einen inneren Schicht (52, 54) und dass die mindestens eine innere Schicht (52, 54) mindestens eine Chromnitrid-(CrN)-Schicht (52) oder mindestens eine Chromcarbonitrid-(CrCN)-Schicht (54) umfasst.

2. Implantat nach Anspruch 1, **dadurch gekennzeichnet, dass** die Hartstoffbeschichtung (44) in Form einer eine Metallionen-Barriere bildenden Beschichtung (44) ausgebildet ist.

3. Implantat nach Anspruch 1 oder 2, **dadurch gekennzeichnet, dass** die Deckschicht (50) direkt eine innere Schicht (52) aus Chromnitrid (CrN) bedeckt.

4. Implantat nach einem der voranstehenden Ansprüche, **dadurch gekennzeichnet, dass** mehrere innere Schichten (52, 54) ausgebildet sind

5. Implantat nach einem der voranstehenden Ansprüche, **dadurch gekennzeichnet, dass** mindestens zwei unterschiedliche innere Schichten (52, 54) ausgebildet sind.

6. Implantat nach Anspruch 5, **dadurch gekennzeichnet, dass** mehr als zwei innere Schichten (52, 54) ausgebildet sind und dass die mindestens zwei unterschiedlichen inneren Schichten (52, 54) abwechselnd übereinander liegend ausgebildet sind.

7. Implantat nach einem der voranstehenden Ansprüche, **dadurch gekennzeichnet, dass** das mindestens eine Implantatteil (10, 22) eine Gelenkkugel oder eine Gelenkpfanne einer Hüftgelenkprothese, eine künstliche Kondyle (10) oder eine Tibiaplatte (22) einer Kniegelenkprothese (20) oder ein eine Gelenkfläche aufweisendes oder tragendes Anlageelement einer Bandscheibenprothese ist.

8. Implantat nach einem der voranstehenden Ansprüche, **dadurch gekennzeichnet, dass** die Zwischenschicht (46) eine metallische Schicht (46) ist.

9. Implantat nach Anspruch 8, **dadurch gekennzeichnet, dass** die Zwischenschicht (46) kein Metall enthält, welches im Implantatmaterial enthalten ist.

10. Implantat nach einem der voranstehenden Ansprüche, **dadurch gekennzeichnet, dass** die Zwischenschicht (46) eine Schicht (46) aus einer Zirkon-Legierung, aus einer Niob-Legierung, aus einer Tantal-Legierung, aus reinem Niob, aus reinem Tantal oder aus reinem Zirkon ist.

11. Implantat nach einem der voranstehenden Ansprüche, **dadurch gekennzeichnet, dass** die Zwischenschicht (46) eine Dicke (56) in einem Bereich von 30·10⁻⁹ m bis 200·10⁻⁹ m aufweist.

12. Verfahren zum Herstellen eines Implantats (20) zum Einsetzen in einen menschlichen oder tierischen Körper, welches mindestens ein Implantatteil (10, 22) umfasst, wobei zur Ausbildung des Implantatteils (10, 22) ein Grundkörper (42) aus einem Implantatmaterial hergestellt wird, welcher eine Oberfläche aufweist, die mindestens teilweise in Form einer künstlichen Gelenkfläche (12, 26) ausgebildet wird, wobei eine verschleißmindernde Hartstoffbeschichtung (44) auf die ganze oder einen Teil der Gelenkfläche (12, 26) aufgebracht wird, wobei vor dem Aufbringen der Hartstoffbeschichtung (44) eine Zwischenschicht (46) zum Vermindern mechanischer Spannungen zwischen der Hartstoffbeschichtung (44) und dem Implantatmaterial auf den mindestens einen Teil der Gelenkfläche (12, 26) aufgebracht wird, wobei die Hartstoffbeschichtung (44) in Form eines mehrschichtigen Schichtsystems ausgebildet wird, **dadurch gekennzeichnet, dass** das Schichtsystem eine vom Implantatteil (10, 22) weg weisende äußere Deckschicht (50) und mindestens eine unter der Deckschicht (50) liegende innere Schicht (52, 54) aufweist, dass eine Härte der Deckschicht (50) größer ist als eine Härte der mindestens einen inneren Schicht (52, 54) und dass die mindestens eine innere Schicht (52, 54) mindestens eine Chromnitrid-(CrN)-Schicht (52) oder mindestens eine Chromcarbonitrid-(CrCN)-Schicht (54) umfasst.

13. Verfahren nach Anspruch 12, **dadurch gekennzeichnet, dass** die Zwischenschicht (46) mit einer Dicke (56) in einem Bereich von 30·10⁻⁹ m bis 200·10⁻⁹ m aufgebracht wird.

14. Verfahren nach Anspruch 12 oder 13, **dadurch gekennzeichnet, dass** als Zwischenschicht (46) eine Schicht (46) aufgebracht wird, die kein Metall enthält, welches im Implantatmaterial enthalten ist.

15. Verfahren nach einem der Ansprüche 12 bis 14, **dadurch gekennzeichnet, dass** als Zwischenschicht (46) eine Schicht aus einer Zirkon-Legierung, aus einer Zirkon enthaltenden Keramik oder aus reinem Zirkon aufgebracht wird.

## Claims

1. Implant (20) for insertion into a human or animal body, the implant comprising at least one implant part (10, 22), wherein the at least one implant part (10, 22) has a base member (42) produced from an implant material with a surface designed at least partially in the form of an artificial joint surface (12, 26), wherein part of or the entire joint surface (12, 26) is covered by a wear-reducing hard material coating (44), wherein an intermediate layer (46) is provided between the hard material coating (44) and the at least one joint surface (12, 26) formed from the implant material for the purpose of reducing tensions between the hard material coating (44) and the implant material, and wherein the hard material coating (44) is designed in the form of a multilayered layer system, **characterized in that** the layer system has an outer covering layer (50) pointing away from the implant part (10, 22) and at least one inner layer (52, 54) located beneath the covering layer (50), **in that** a hardness of the covering layer (50) is greater than a hardness of the at least one inner layer (52, 54), and **in that** the at least one inner layer (52, 54) comprises at least one chromium nitride (CrN) layer (52) or at least one chromium carbonitride (CrCN) layer (54).

2. Implant in accordance with claim 1, **characterized in that** the hard material coating (44) is designed in the form of a coating (44) forming a metal ion barrier.

3. Implant in accordance with claim 1 or 2, **characterized in that** the covering layer (50) directly covers an inner layer (52) consisting of chromium nitride (CrN).

4. Implant in accordance with any one of the preceding claims, **characterized in that** several inner layers (52, 54) are formed.

5. Implant in accordance with any one of the preceding claims, **characterized in that** at least two different inner layers (52, 54) are formed.

6. Implant in accordance with claim 5, **characterized in that** more than two inner layers (52, 54) are formed, and **in that** the at least two different inner layers (52, 54) are designed to be superimposed alternatingly.

7. Implant in accordance with any one of the preceding claims, **characterized in that** the at least one implant part (10, 22) is a joint ball or a joint socket of a hip joint prosthesis, an artificial condyle (10) or a tibial plate (22) of a knee joint prosthesis (20) or a contact element, having or bearing a joint surface, of an intervertebral disk prosthesis.

8. Implant in accordance with any one of the preceding claims, **characterized in that** the intermediate layer (46) is a metallic layer (46).

9. Implant in accordance with claim 8, **characterized in that** the intermediate layer (46) contains no metal that is contained in the implant material.

10. Implant in accordance with any one of the preceding claims, **characterized in that** the intermediate layer (46) is a layer (46) consisting of a zirconium alloy, of a niobium alloy, of a tantalum alloy, of pure niobium, of pure tantalum or of pure zirconium.

11. Implant in accordance with any one of the preceding claims, **characterized in that** the intermediate layer (46) has a thickness (56) in a range of 30 x 10⁻⁹ m to 200 x 10⁻⁹ m.

12. Method for the production of an implant (20) for insertion into a human or animal body, the implant comprising at least one implant part (10, 22), wherein a base member (42) is produced from an implant material to form the implant part (10, 22), the base member (42) having a surface designed at least partially in the form of an artificial joint surface (12, 26), wherein a wear-reducing hard material coating (44) is applied to the entire or a part of the joint surface (12, 26), wherein prior to the application of the hard material coating (44) an intermediate layer (46) is applied to the at least one part of the joint surface (12, 26) for the purpose of reducing mechanical tensions between the hard material coating (44) and the implant material, wherein the hard material coating (44) is designed in the form of a multilayered layer system, **characterized in that** the layer system has an outer covering layer (50) pointing away from the implant part (10, 22) and at least one inner layer (52, 54) located beneath the covering layer (50), **in that** a hardness of the covering layer (50) is greater than a hardness of the at least one inner layer (52, 54), and **in that** the at least one inner layer (52, 54) comprises at least one chromium nitride (CrN) layer (52) or at least one chromium carbonitride (CrCN) layer (54).

13. Method in accordance with claim 12, **characterized in that** the intermediate layer (46) is applied with a thickness (56) in a range of 30 x 10⁻⁹ m to 200 x 10⁻⁹ m.

14. Method in accordance with claim 12 or 13, **characterized in that** a layer (46) containing no metal that is contained in the implant material is applied as intermediate layer (46).

15. Method in accordance with any one of claims 12 to 14, **characterized in that** a layer consisting of a zirconium alloy, a ceramic containing zirconium or of pure zirconium is applied as intermediate layer (46).

## Revendications

1. Implant (20) destiné à être implanté dans un corps humain ou animal, et comprenant au moins une pièce d'implant (10, 22), implant
dans lequel ladite au moins une pièce d'implant (10, 22) comporte un corps de base (42) fabriqué en un matériau d'implant et présentant une surface qui est configurée au moins en partie sous la forme d'une surface articulaire artificielle (12, 26),
dans lequel une partie ou la totalité de la surface articulaire (12, 26) est recouverte d'un revêtement de matériau dur (44) permettant de réduire l'usure,
dans lequel entre le revêtement de matériau dur (44) et ladite au moins une surface articulaire (12, 26) constituée du matériau d'implant, est prévue une couche intermédiaire (46) destinée à diminuer les contraintes entre le revêtement de matériau dur (44) et le matériau d'implant, et
dans lequel le revêtement de matériau dur (44) est réalisé sous la forme d'un système de couche à couches multiples,
**caractérisé en ce que** le système de couche présente une couche de recouvrement extérieure (50) sur le côté éloigné de la pièce d'implant (10, 22), et au moins une couche intérieure (52, 54) située sous la couche de recouvrement (50), **en ce qu'**une dureté de la couche de recouvrement (50) est supérieure à une dureté de ladite au moins une couche intérieure (52, 54), et **en ce que** ladite au moins une couche intérieure (52, 54) comprend au moins une couche (52) de nitrure de chrome (CrN) ou au moins une couche (54) de carbonitrure de chrome (CrCN).

2. Implant selon la revendication 1, **caractérisé en ce que** le revêtement de matériau dur (44) est réalisé sous la forme d'un revêtement (44) formant une barrière d'ions métalliques.

3. Implant selon la revendication 1 ou la revendication 2, **caractérisé en ce que** la couche de recouvrement (50) recouvre directement une couche intérieure (52) de nitrure de chrome (CrN).

4. Implant selon l'une des revendications précédentes, **caractérisé en ce que** sont réalisées plusieurs couches intérieures (52, 54).

5. Implant selon l'une des revendications précédentes, **caractérisé en ce que** sont réalisées au moins deux couches intérieures (52, 54) différentes.

6. Implant selon la revendication 5, **caractérisé en ce que** sont réalisées plus de deux couches intérieures (52, 54), et **en ce que** les lesdites au moins deux couches intérieures (52, 54) différentes sont prévues en étant superposées de manière alternée.

7. Implant selon l'une des revendications précédentes, **caractérisé en ce que** ladite au moins une pièce d'implant (10, 22) est une rotule d'articulation ou une cupule cotyloïdienne d'une prothèse de l'articulation de la hanche, un condyle artificiel (10) ou un plateau tibial (22) d'une prothèse (20) de l'articulation du genou, ou un élément d'appui présentant ou portant une surface articulaire d'une prothèse de disque intervertébral.

8. Implant selon l'une des revendications précédentes, **caractérisé en ce que** la couche intermédiaire (46) est une couche métallique (46).

9. Implant selon la revendication 8, **caractérisé en ce que** la couche intermédiaire (46) ne renferme pas de métal qui serait contenu dans le matériau d'implant.

10. Implant selon l'une des revendications précédentes, **caractérisé en ce que** la couche intermédiaire (46) est une couche (46) en un alliage de zirconium, en un alliage de niobium, en un alliage de tantale, en niobium pur, en tantale pur ou en zirconium pur.

11. Implant selon l'une des revendications précédentes, **caractérisé en ce que** la couche intermédiaire (46) présente une épaisseur (56) située dans une plage de 30·10⁻⁹ m à 200·10⁻⁹ m.

12. Procédé de fabrication d'un implant (20) destiné à être implanté dans un corps humain ou animal, et comprenant au moins une pièce d'implant (10, 22), procédé
d'après lequel pour réaliser ladite au moins une pièce d'implant (10, 22), on fabrique un corps de base (42) en un matériau d'implant, qui présente une surface qui est configurée au moins en partie sous la forme d'une surface articulaire artificielle (12, 26),
d'après lequel on applique un revêtement de matériau dur (44) permettant de réduire l'usure, sur une partie ou sur la totalité de la surface articulaire (12, 26), d'après lequel, avant l'application du revêtement de matériau dur (44), on applique une couche intermédiaire (46) destinée à diminuer les contraintes mécaniques entre le revêtement de matériau dur (44) et le matériau d'implant, sur ladite au moins une partie de la surface articulaire (12, 26),
et d'après lequel on réalise le revêtement de matériau dur (44) sous la forme d' un système de couche à couches multiples,
**caractérisé en ce que** le système de couche présente une couche de recouvrement extérieure (50) sur le côté éloigné de la pièce d'implant (10, 22), et au moins une couche intérieure (52, 54) située sous la couche de recouvrement (50), **en ce qu'**une dureté de la couche de recouvrement (50) est supérieure à une dureté de ladite au moins une couche intérieure (52, 54), et **en ce que** ladite au moins une couche intérieure (52, 54) comprend au moins une couche (52) de nitrure de chrome (CrN) ou au moins une couche (54) de carbonitrure de chrome (CrCN).

13. Procédé selon la revendication 12, **caractérisé en ce que** la couche intermédiaire (46) est appliquée avec une épaisseur (56) située dans une plage de 30·10⁻⁹ m à 200·10⁻⁹ m_{.}

14. Procédé selon la revendication 12 ou la revendication 13, **caractérisé en ce qu'**en guise de couche intermédiaire (46), on applique une couche (46) ne renfermant pas de métal qui serait contenu dans le matériau d'implant.

15. Procédé selon l'une des revendications 12 à 14, **caractérisé en ce qu'**en guise de couche intermédiaire (46), on applique une couche en un alliage de zirconium, en une céramique renfermant du zirconium ou en zirconium pur.
